# EUROPÄISCHE PATENTSCHRIFT

(11) **EP 2 147 316 B1**
(45) Veröffentlichungstag und Bekanntmachung des Hinweises auf die Patenterteilung: **10.07.2013**
(21) Anmeldenummer: 08758039.5
(22) Anmeldetag: 08.05.2008
(51) Int. Cl.: G01N 33/74

(54) **DIAGNOSE UND RISIKOSTRATIFIZIERUNG MITTELS NT-PROET-1**
DIAGNOSIS AND RISK STRATIFICATION USING NT-proET-1
DIAGNOSTIC ET STRATIFICATION DU RISQUE AU MOYEN DE NT-proET-1

(30) Priorität: 08.05.2007 DE 102007021443
(43) Veröffentlichungstag der Anmeldung: 27.01.2010
(60) Teilanmeldung: 10191423.2
(73) Patentinhaber: B.R.A.H.M.S GmbH, 16761 Hennigsdorf (DE)
(72) Erfinder: BERGMANN, Andreas, 12351 Berlin (DE); STRUCK, Joachim, 13465 Berlin (DE)
(74) Vertreter: Simandi, Claus
(86) Internationale Anmeldenummer: PCT/DE2008/000781
(87) Internationale Veröffentlichungsnummer: WO 2008/135038

(56) Entgegenhaltungen:
- EP-A- 1 564 558
- STRUCK ET AL: "Proteolytic processing pattern of the endothelin-1 precursor in vivo" PEPTIDES, ELSEVIER, AMSTERDAM, Bd. 26, Nr. 12, 1. Dezember 2005 (2005-12-01), Seiten 2482-2486, XP005174851 ISSN: 0196-9781 in der Anmeldung erwähnt
- PAPASSOTIRIOU JANA ET AL: "Immunoluminometric assay for measurement of the C-terminal endothelin-1 precursor fragment in human plasma" CLINICAL CHEMISTRY, Bd. 52, Nr. 6, Juni 2006 (2006-06), Seiten 1144-1151, XP002484207 ISSN: 0009-9147
- GIAID ADEL ET AL: "Expression of endothelin-1 in the lungs of patients with pulmonary hypertension" NEW ENGLAND JOURNAL OF MEDICINE, Bd. 328, Nr. 24, 1993, Seiten 1732-1739, XP008098918 ISSN: 0028-4793
- STEWART D J ET AL: "INCREASED PLASMA ENDOTHELIN-1 IN PULMONARY HYPERTENSION MARKER OR MEDIATOR OF DISEASE" ANNALS OF INTERNAL MEDICINE, Bd. 114, Nr. 6, 1991, Seiten 464-469, XP008098981 ISSN: 0003-4819
- HUMBERT MARC ET AL: "Cellular and molecular pathobiology of pulmonary arterial hypertension" JOURNAL OF THE AMERICAN COLLEGE OF CARDIOLOGY, Bd. 43, Nr. 12, Suppl. S, 16. Juni 2004 (2004-06-16), Seiten 13S-24S, XP008098866 ISSN: 0735-1097

### SEQUENCE LISTING

<110> Brahms Aktiengesellschaft
<120> Diagnose und Risikostratifizierung mittels NT-proET-1
<130> 03/07 BRAHMS 35WO
<140> PCT/DE2008/
   <141> 2008-05-08
<150> 10 2007 041 443.1
   <151> 2007-05-08
<160> 6
<170> PatentIn version 3.3
<210> 1
   <211> 212
   <212> PRT
   <213> Human
<400> 1
   Met Asp Tyr Leu Leu Met Ile Phe Ser Leu Leu Phe Val Ala Cys Gln
   1               5                   10                  15
   Gly Ala Pro Glu Thr Ala Val Leu Gly Ala Glu Leu Ser Ala Val Gly
               20                  25                  30
   Glu Asn Gly Gly Glu Lys Pro Thr Pro Ser Pro Pro Trp Arg Leu Arg
           35                  40                  45
   Arg Ser Lys Arg Cys Ser Cys Ser Ser Leu Met Asp Lys Glu Cys Val
       50                  55                  60
   Tyr Phe Cys His Leu Asp Ile Ile Trp Val Asn Thr Pro Glu His Val
   65                  70                  75                  80
   Val Pro Tyr Gly Leu Gly Ser Pro Arg Ser Lys Arg Ala Leu Glu Asn
                   85                  90                  95
   Leu Leu Pro Thr Lys Ala Thr Asp Arg Glu Asn Arg Cys Gln Cys Ala
               100                 105                 110
   Ser Gln Lys Asp Lys Lys Cys Trp Asn Phe Cys Gln Ala Gly Lys Glu
           115                 120                 125
   Leu Arg Ala Glu Asp Ile Met Glu Lys Asp Trp Asn Asn His Lys Lys
       130                 135                 140
   Gly Lys Asp Cys Ser Lys Leu Gly Lys Lys Cys Ile Tyr Gln Gln Leu
   145                 150                 155                 160
   Val Arg Gly Arg Lys Ile Arg Arg Ser Ser Glu Glu His Leu Arg Gln
                   165                 170                 175
   Thr Arg Ser Glu Thr Met Arg Asn Ser Val Lys Ser Ser Phe His Asp
   180 185 190
   Pro Lys Leu Lys Gly Lys Pro Ser Arg Glu Arg Tyr Val Thr His Asn
   195 200 205
   Arg Ala His Trp
   210
<210> 2
   <211> 14
   <212> PRT
   <213> Human
<400> 2
   Ala Pro Glu Thr Ala Val Leu Gly Ala Glu Leu Ser Ala Val
   1 5 10
<210> 3
   <211> 21
   <212> PRT
   <213> Human
<400> 3
   Gly Glu Asn Gly Gly Glu Lys Pro Thr Pro Ser Pro Pro Trp Arg Leu
   1 5 10 15
   Arg Arg Ser Lys Arg
   20
<210> 4
   <211> 15
   <212> PRT
   <213> Artificial
<220>
   <223> synthetic derivat
<400> 4
   Cys Ala Pro Glu Thr Ala Val Leu Gly Ala Glu Leu Ser Ala Val
   1 5 10 15
<210> 5
   <211> 22
   <212> PRT
   <213> artificial
<220>
   <223> synthetic derivat
<400> 5
   Gly Glu Asn Gly Gly Glu Lys Pro Thr Pro Ser Pro Pro Trp Arg Leu
   1 5 10 15
   Arg Arg Ser Lys Arg Cys
   20
<210> 6
   <211> 35
   <212> PRT
   <213> Human
<400> 6
   Ala Pro Glu Thr Ala Val Leu Gly Ala Glu Leu Ser Ala Val Gly Glu
   1 5 10 15
   Asn Gly Gly Glu Lys Pro Thr Pro Ser Pro Pro Trp Arg Leu Arg Arg
   20 25 30
   Ser Lys Arg
   35

## Beschreibung

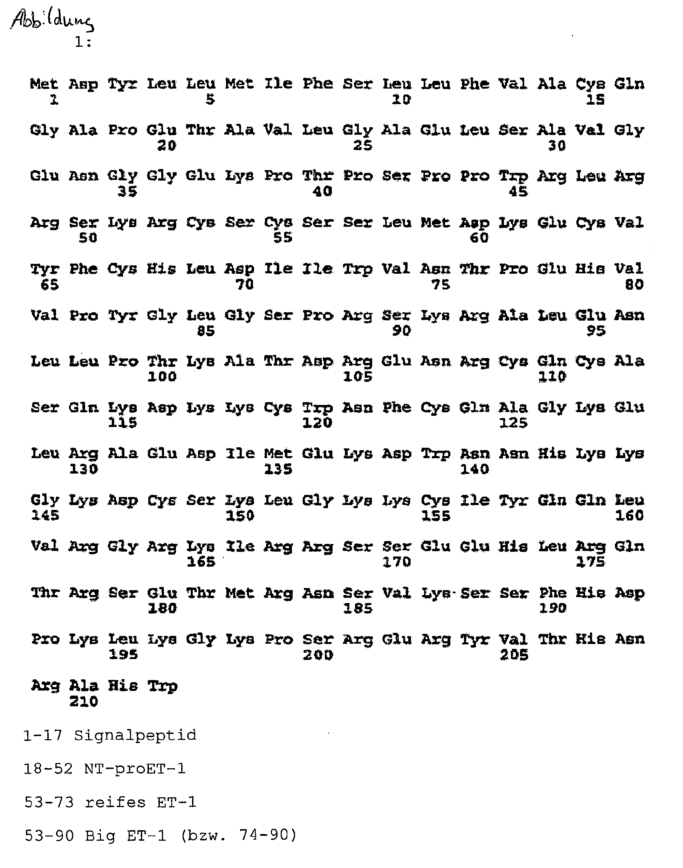

Die Erfindung betrifft ein Verfahren zur Diagnose und / oder Risikostratifizierung von Herzerkrankungen wobei eine Bestimmung des freien Fragment N-terminales proEndothelin (NT-proET-1; AS 18-52 des preproET gemäß Figur 1) oder Fragmenten davon, erfolgt.

Zwecks einer geeigneten Therapie, bedarf es einer frühen Diagnose und Differenzierung von Herzerkrankungen bereits in der Notaufnahme in Verbindung mit der Notwendigkeit klinische Entscheidungen zu treffen.

Im Stand der Technik ist das reife ET-1 (21 AS) zur Diagnostik samt Vorläuferprotein Big-ET 1 (38AS, siehe Figur 1) beschrieben (Rossi GP, Seccia TM, Albertin G, Pessina AC. Measurement of endothelin: clinical and research use. Ann Clin Biochem 2000;37(Pt 5):608-26.2; Aubin P, Le Brun G, Moldovan F, Villette JM, Creminon C, Dumas J, et al. Sandwich-type enzyme immunoassay for big endothelin-I in plasma: concentrations in healthy human subjects unaffected by sex or posture. Clin Chem 1997;43: 64-70). Ferner ist bekannt, dass Endothelin-Prohormone zur Diagnose von Sepsis in WO 00/22439 bzw. EP 1121600 B1 der Anmelderin offenbart sind.

Ferner sind die C-terminalen ProEndothelin Fragmente (CT-proET-1) mit den Aminosäuresequenzen 93-212 oder 168-212 des preproEndothelin (Figur 1, SEQ ID No. 1) zur Diagnose von Herz-Kreislauferkrankungen zur indirekten Bestimmung des Endothelin-1 oder des big - Endothelin-1 Gehaltes in EP 1564558 B1 beschrieben.

Struck et al. (2005) Peptides, Vol. 26, Seiten 2482-2486 offenbart die Messung des Vorläuferpeptids von Endothelin-1 in Plasma. Dies stellt jedoch keinen Zusammenhang des Peptids als Marker für die Diagnose oder Stratifizierung von Herzkrankheiten her. Ferner werden Antikörper offenbart, die an die Epitope von 18-53 binden können.

Papassotiriou Jana et al. (2006) Clinical Chemistry, Vol. 52, Seiten 1144-1151 betrifft die diagnostische Eignung von "CT-proET-1" und berichtet ausschließlich über "Peptide aus ET-1 precursor". NT-proET-1 wird nicht ausdrücklich erwähnt. Ferner wird erläutert, dass diese "Peptide aus ET-1 precursor" zur indirekten Bestimmung von freigesetzten Mengen ET-1 geeignet sind. Papassotiriou Jana et al. beschreiben weder explizit NT-proET-1 noch, dass NT-proET-1 eine eigenständige und unabhängige diagnostische Funktion zukommt. Es findet sich auch kein Hinweis, dass mittels NT-proET-1 eine Diagnose und Risikostratifizierung von Herzerkrankungen, unabhängig von ET-1 und bigET-1, erfolgen kann.

Nachteilig an den bekannten Diagnoseverfahren unter Verwendung der bisher bekannten Marker ist jedoch weiterhin, dass eine frühzeitige und vollständige Erfassung von Risikopatienten nicht ausreichend gelingt und daher eine Risikostratifizierung nicht ausreichend erfolgt. Eine der Erfindung zugrunde liegende Aufgabe besteht daher darin, ein Verfahren zur Risikostratifizierung von Herzerkrankungen zu entwickeln, das eine verbesserte Erfassung von Risikopatienten ermöglicht.

Ferner ist nachteilig, dass im Stand der Technik zumeist keine hinreichende Sensitivität und/oder Spezifität der Marker erreicht wird.

Eine weitere Aufgabe besteht daher darin, ein Verfahren zur Risikostratifizierung von Herzerkrankungen bereitzustellen, wobei mindestens ein Marker oder eine Kombination von Markern eine hinreichende Sensitivität und Spezifität in einer in-vitro Diagnose aufweist.

Daher ist es Aufgabe der vorliegenden Erfindung ein Verfahren zur Diagnose und / oder Risikostratifizierung von Herzerkrankungen bereitzustellen.

Die Aufgabe wird durch ein Verfahren zur Diagnose und Risikostratifizierung von Herzerkrankungen gelöst, wobei eine Bestimmung des N-terminales proEndothelin (kurz: "NT-proET-1") oder Fragmenten davon, erfolgt (nachstehend erfindungsgemäßes Verfahren).

Gegenstand der Erfindung ist ein Verfahren zur in-vitro Diagnose und oder Risikostratifizierung von Herzerkrankungen, dadurch gekennzeichnet, dass eine Bestimmung des freien Fragments NT-proET-1 (18-52) oder von Fragmenten von, in einer Körperflüssigkeitsprobe von einem zu untersuchenden Patienten erfolgt.

Die Erfindung betrifft weiterhin die Verwendung des freien Fragments NT-proET-1 oder von Fragmenten davon, zur Diagnose und / oder Risikostratifizierung von Herzerkrankungen und ggfs. weiterer Marker.

Die Erfindung betrifft weiterhin die Verwendung eines Kits zur Diagnose und / oder Risikostratifizierung von Herzerkrankungen enthaltend Nachweisreagenzien zur Bestimmung des freien Fragments NT-proET-1 oder von Fragmenten davon, und ggfs. weitere Marker.

Überraschender Weise weisen NT-proET-1 oder Fragmente und Teilpeptide davon, eine hohe Sensitivität und Spezifität für die Diagnose von Herzerkrankungen auf (Siehe Beispiele und Figuren).

Der erfindungsgemäße Begriff "Herzerkrankungen" umfasst insbesondere das "akute Koronarsyndrom" und dessen verschiedene Phasen der koronaren Herzerkrankung, die unmittelbar lebensbedrohlich sind. Dies betrifft insbesondere die Notfallmedizin, und zwar einen akuten Myokardinfarkt und oder Angina pectoris sowie einen plötzlichen Herztod. Neben dem akuten Myokardinfarkt, der nach WHO-Kriterien (WHO (1979): Nomenclature and criteria for diagnosis of ischemic heart disease. Report of the Joint International Society and Federation of Cardiology/World Health Organization task force on standardization of clinical nomenclature, Circulation 59 (3): 607-609) definiert ist als akutes, länger als 20 Minuten andauerndes Brustschmerzereignis, verbunden mit ST-Streckenhebungen und/oder einer Erhöhung myokardialer Enzyme, wurde der Begriff der instabilen Angina pectoris (AP) geprägt, die erfindungsgemäß unter "Herzerkrankungen" zu lesen sind (Hamm CW: Leitlinien: Akutes Koronarsyndrom (ACS) - Teil 1: ACS ohne persistierende ST-Hebung. Z Kardiol (2004) 93:72-90).

Im Rahmen dieser Erfindung wird unter "Myokardinfarkt" (Herzinfarkt, AMI (acute myocardial infarction)) eine akute und lebensbedrohliche Erkrankung des Herzens verstanden, wobei ein Absterben oder Gewebsuntergang (Infarkt) von Teilen des Herzmuskels (Myokard) erfolgt, infolge einer Durchblutungsstörung (Ischämie), die in der Regel länger als 20 Minuten besteht. Leitsymptom des Herzinfarktes ist ein plötzlich auftretender, mehr als 20 Minuten anhaltender und meist starker Schmerz im Brustbereich ("chest pain"), der in die Schultern, Arme, Unterkiefer und Oberbauch ausstrahlen kann und von Schweißausbrüchen, Übelkeit und evtl. Erbrechen begleitet sein kann. Infolge eines Myokardinfarktes ist ein Herzversagen möglich.

Der Begriff "post-Myokardinfarkt" bedeutet, dass ein Patient bereits einen Myokardinfarkt in der Vergangenheit, d.h. z.B. mehr als 1 Stunde, insbesondere 20 Stunden, insbesondere 1 bis 5 Tage oder 3 bis 5 Tage erlitten hat und nunmehr in der Post ("Danach") - Phase lebt und keine unmittelbare Todesfolge erlitten hat, jedoch mittelbar und unmittelbar mit einem weiteren nachteiligen Ereignis gerechnet werden kann, wie ein weiterer (Folge-) Myokardinfarkt, Herzversagen oder Todeseintritt oder sonstige Verschlechterung des Patienten.

Im Rahmen dieser Erfindung wird unter "Herzerkrankungen" ebenfalls die "Herzinsuffizienz" subsumiert. "Herzinsuffizienz" ist ein akutes oder chronisches Unvermögen des Herzens, die Gewebe mit ausreichend Blut und infolge dessen genügend Sauerstoff zu versorgen, um den Gewebestoffwechsel in Ruhe oder unter Belastung sicherzustellen. Klinisch liegt eine Herzinsuffizienz vor, wenn typische Symptome (Dyspnoe, Müdigkeit, Flüssigkeitsretention) bestehen, denen ursächlich eine kardiale Funktionsstörung im Sinne einer systolischen oder diastolischen Funktionsstörung zugrunde liegt. Ebenfalls die chronische Herzinsuffizienz (CHF) ist erfindungsgemäß umfasst(Kardiologie compact, herausgegeben von Chrisian Mewis, Reimer Riessen und Ioakim Spyridopoulos, 2. unveränderte Auflage, Thieme 2006). Ursachen einer Herzinsuffizienz können sein: Herzklappenfehler (z. B. als Spätfolge des rheumatischen Fiebers), Myokarditis (Herzmuskelentzündung), Herzrhythmusstörungen, Herzinfarkt neben zu hohem Blutdruck (Hypertonie) und/oder Arteriosklerose (Verkalkung) der Herzkranzgefäße (koronare Herzkrankheit). Weiterhin erfindungsgemäß umfasst sind hypertensive Herzkrankheit mit (kongestiver) Herzinsuffizienz, Hypertensive Herz- und Nierenkrankheit mit (kongestiver) Herzinsuffizienz, Primäre Rechtsherzinsuffizienz, Sekundäre Rechtsherzinsuffizienz, Linksherzinsuffizienz ohne Beschwerden (NYHA-Stadium I), Linksherzinsuffizienz mit Beschwerden bei stärkerer Belastung (NYHA-Stadium II), Linksherzinsuffizienz mit Beschwerden bei leichterer Belastung (NYHA-Stadium III), Linksherzinsuffizienz mit Beschwerden in Ruhe (NYHA-Stadium IV) und kardiogener Schock.

Alle genannten Indikationen werden zudem z.B. im Pschyrembel, De Gruyter, 9. Auflage, Berlin 2004 beschrieben.

Der Begriff "Risikostratifizierung" umfasst erfindungsgemäß das Auffinden von Patienten, insbesondere Notfallpatienten und Risikopatienten, mit der schlechteren Prognose, zwecks intensiverer Diagnostik und Therapie/Behandlung von Herzerkrankungen, insbesondere des akuten Koronarsyndroms sowie Myokardinfarkt, Angina pectoris und / oder post-Myokardinfarkt, Herzinsuffizienz mit dem Ziel einen möglichst günstigen Verlauf der Krankheit zu ermöglichen. Eine erfindungsgemäße Risikostratifizierung erlaubt in Folge ein effektives Behandlungsverfahren, die beispielsweise beim akuten Koronarsyndrom mit den perkutanen Koronarinterventionen und den neueren Arzneimitteln gegeben sind oder zur Behandlung oder Therapie von Herzinsuffizienz, solche wie ACE-Hemmer, AT1-Antagonisten: Blocker des Angiotensin-II-Rezeptors (Subtyp 1), Betablocker Bisoprolol, Carvedilol, Metoprolol und Nebivolol, Vasopressin-Rezeptor-Antagonisten, Aldosteronantagonisten ab NYHA-Stadium III, Kalzium-Sensitizer (Levosimendan).

Daher betrifft die Erfindung ebenfalls die Identifizierung von Patienten mit erhöhtem Risiko oder/und einer ungünstigen Prognose von Herzerkrankungen und zwar bei symptomatischen und / oder asymptomatischen Patienten, insbesondere Notfallpatienten.

Besonders vorteilhaft kann insbesondere in Fällen der Notfall- und /oder Intensivmedizin mittels des erfindungsgemäßen Verfahren eine sichere Stratifizierung erfolgen. Das erfindungsgemäße Verfahren ermöglicht daher klinische Entscheidungen, die zu einem schnellen Therapieerfolg und zur Vermeidung von Todesfällen führen. Solche klinischen Entscheidungen umfassen ebenfalls weiterführende Behandlung mittels Arzneimitteln zur Behandlung oder Therapie von Herzerkrankungen.

Daher betrifft die Erfindung ebenfalls ein Verfahren zur Diagnose und / oder Risikostratifizierung von Patienten von Herzerkrankungen zur Durchführung von klinischen Entscheidungen, wie weiterführende Behandlung und Therapie mittels Arzneimitteln, vorzugsweise in der zeitlich kritischen Intensivmedizin oder Notfallmedizin, einschließlich der Entscheidung zur Hospitalisierung des Patienten.

In einer weiteren bevorzugten Ausführungsform betrifft das erfindungsgemäße Verfahren daher die Therapiesteuerung von Herzerkrankungen

In einer weiteren bevorzugten Ausführungsform des erfindungsgemäßen Verfahrens erfolgt die zur Diagnose und /oder Risikostratifizierung zur Prognose, zur differentialdiagnostischen Früherkennung und Erkennung, zur Beurteilung des Schweregrades und zur therapiebegleitenden Verlaufsbeurteilung.

In einer weiteren bevorzugten Ausführungsform betrifft die Erfindung ein Verfahren zur in-vitro Diagnostik zur Früh- oder Differentialdiagnose oder Prognose von Herzerkrankungen, wobei eine Bestimmung des Marker NT-proET-1 oder Fragmente davon, an einem zu untersuchenden Patienten durchgeführt wird.

In einer Ausführungsform des erfindungsgemäßen Verfahrens wird dem zu untersuchenden Patienten Körperflüssigkeit, vorzugsweise Blut entnommen, wahlweise Vollblut oder Serum oder erhältliches Plasma und die Diagnose erfolgt *in vitro*/*ex vivo,* d.h. außerhalb des menschlichen oder tierischen Körpers. Aufgrund der Bestimmung des Markers NT-proET-1 oder Fragmenten und Teilpeptiden davon, wird eine hohe Sensitivität und Spezifität erzielt (siehe Beispiele und Figuren) und anhand der vorhandenen Menge in mindestens einer Patientenprobe kann die Diagnose oder Risikostratifizierung erfolgen.

Im Rahmen dieser Erfindung wird unter "NT-proET-1" ein humanes Protein oder Polypeptid verstanden, das aus dem Preproendothelin erhalten werden kann und im Rahmen des Preproendothelins (SEQ ID No.1 und Figur 1) die freien Fragmente mit den Aminosäuren 18-52 umfassen und die Fragmente APETAVLGAELSAV (SEQ ID No. 2) Pos. 18-31 von preproET-1 (SEQ ID No.1 und Figur 1) und GENGGEKPTPSPPWRLRRSKR (SEQ ID No. 3) Pos. 32-52 von preproET-1 (SEQ ID No.1 und Figur 1). Ferner können diese erfindungsgemäßen Polypeptide posttranslationale Modifikationen aufweisen, wie Glykolisierung, Lip(o)idisierung oder Derivatisierungen.

In einer weiteren Ausführungsform kann die Bestimmung von NT-proET-1 oder Fragmenten und Teilpeptiden davon, zusätzlich mit weiteren Markern erfolgen und zwar vorzugsweise solche, die bereits auf eine Herzerkrankung, insbesondere koronare Herzerkrankung hinweisen.

Daher betrifft die Erfindung eine solche Ausführungsform des erfindungsgemäßen Verfahrens, wobei die Bestimmung zusätzlich mit mindestens einem weiteren Marker ausgewählt aus der Gruppe inflammatorischer Marker, cardiovaskulärer Marker, neurohormonaler Marker oder ischämischer Marker an einem zu untersuchenden Patienten durchgeführt wird.

Erfindungsgemäß kann der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt sein sowie der cardiovaskuläre Marker, insbesondere ein Nekrose von Herzmuskelgewebe anzeigender Marker und den Blutdruck beeinflussender Marker aus mindestens einem Marker aus der Gruppe Kreatinkinase, Myoglobin, Myeloperoxidase, natriuretisches Protein, insbesondere ANP (bzw. ANF), proANP, NT-proANP, BNP, proBNP, NT-proBNP oder jeweils eine Teilsequenz davon, kardiale Troponin, CRP ausgewählt sein. Ferner werden hierunter ebenfalls Kreislaufregulierende (Pro)Hormone verstanden, insbesondere wie pro-Gastrin-Releasing Peptid (proGRP), pro-Endothelin-2, pro-Endothelin-3, pro-Leptin, pro-Neuropeptid-Y, pro-Somatostatin, pro-Neuropeptid-YY, pro-Opiomelanocortin, pro-Adrenomedullin (proADM), pro-Vasopressin (proAVP) oder jeweils eine Teilsequenz davon.

Der ischämische Marker kann aus mindestens einem Marker aus der Gruppe Troponin I und T, CK-MB ausgewählt sein. Darüber hinaus kann der neurohormonale Marker mindestens ein weiteres natriuretisches Protein sein, insbesondere ANP (bzw. ANF), proANP, NT-proANP, BNP, proBNP oder jeweils eine Teilsequenz davon.

In einer weiteren Ausführungsform der Erfindung kann das erfindungsgemäße Verfahren im Rahmen einer in-vitro Diagnose mittels parallelen oder simultanen Bestimmungen der Marker durchgeführt werden (z.B. Multititerplatten mit 96 und mehr Kavitäten), wobei die Bestimmungen an mindestens einer Patientenprobe durchgeführt werden.

Ferner kann das erfindungsgemäße Verfahren und dessen Bestimmungen in einer diagnostischen Vorrichtung anhand eines Analyseautomaten, insbesondere mittels einem Kryptor (http://www.kryptor.net/) durchgeführt werden.

In einer weiteren Ausführungsform kann das erfindungsgemäße Verfahren und dessen Bestimmungen mittels einem Schnelltest durchgeführt werden (z.B. lateral-flow Test oder Point-of Care), sei es in Einzel- oder Multiparameterbestimmung. In einer besonders bevorzugten Ausführungsform handelt es sich um einen Selbsttest oder um eine Vorrichtung, die in der Notfalldiagnostik geeignet ist.

Ferner betrifft die Erfindung die Verwendung von NT-proET-1 oder Fragmente davon, zur Risikostratifizierung von Herzerkrankungen und/oder zur in-vitro Diagnostik zur Früh- oder Differentialdiagnose oder Prognose von Herzerkrankungen.

Eine weitere Aufgabe ist die Verwendung einer entsprechenden diagnostischen Vorrichtung zur Durchführung des erfindungsgemäßen Verfahrens.

Im Rahmen dieser Erfindung wird unter einer solchen diagnostischen Vorrichtung, insbesondere ein Array oder Assay verstanden (z.B. Immunoassay, ELISA etc.), im weitesten Sinne eine Vorrichtung zur Durchführung des erfindungsgemäßen Verfahrens.

Die Erfindung betrifft zudem die Verwendung eines Kits zur Diagnose und / oder Risikostratifizierung von Herzerkrankungen enthaltend Nachweisreagenzien zur Bestimmung von NT-proET-1 oder Fragmenten davon, ggfs. oben genannte weitere Marker. Solche Nachweisreagenzien umfassen z.B. Antikörper etc.

Nachfolgende Beispiele und Figuren dienen zur näheren Erläuterung der Erfindung, jedoch ohne die Erfindung auf diese Beispiele und Figuren zu beschränken.

### Beispiele und Abbildnugen:

### Immunoassay

Es wurde ein Sandwichimmunoassay zur. Messung von NT-proET-1 wie beschrieben in Struck et al (Struck J, Morgenthaler NG, Bergmann A. Proteolytic processing pattern of the endothelin-1 precursor in vivo. Peptides 2005; 26:2482-6) verwendet.

### Peptidsynthesen:

Abgeleitet von der bekannten Aminosäuresequenz von preproET-1 (Figur 1) wurden zwei Bereiche ausgewählt (Pos. 18-31, 32-52).

Die Bereiche wurden, in einem Fall ergänzt um einen N-terminalen Cystein-Rest, nach Standardverfahren als lösliche Peptide chemisch synthetisiert, gereinigt, mittels Massenspektrometrie und Reversed Phase HPLC qualitätskontrolliert und in Aliquots lyophilisiert (Firma JPT, Berlin, Deutschland). Die Aminosäuresequenzen der Peptide lauten:
PAV15 CAPETAVLGAELSAV Pos. 18-31 von preproET-1 (Figur 1), MPGC22 GENGGEKPTPSPPWRLRRSKRC Pos. 32-52 von preproET-1 (Figur 1).

Des Weiteren wurde ein Peptid synthetisiert, das den Bereich Pos. 18-52 von preproET-1 umfasst (PAR35 APETAVLGAELSAVGENGGEKPTPSPPWRLRRSKR).

### Konjugation und Immunisierung

Mittels MBS (m-Maleimidobenzoyl-N-hydroxysuccinimid Ester) wurden die Peptide PAV15 und MPGC22 an das Trägerprotein KLH (Keyhole limpet hemocyanin) konjugiert (s. Arbeitsanleitung "NHS-Esters-Maleimide Crosslinkers" Firma PIERCE, Rockford, IL, USA). Mit diesen Konjugaten wurden Schafe nach folgendem Schema immunisiert: Jedes Schaf erhielt initial 100 µg Konjugat (Massenangabe bezogen auf den Peptid-Anteil des Konjugats) und anschließend 4-wöchentlich je 50 µg Konjugat (Massenangabe bezogen auf den Peptid-Anteil des Konjugats). Beginnend mit dem vierten Monat nach Beginn der Immunisierung wurden 4-wöchentlich je Schaf 700 ml Blut abgenommen und daraus durch Zentrifugation Antiserum gewonnen. Konjugationen, Immunisierungen und Gewinnung von Antiseren wurden von der Firma MicroPharm, Carmarthenshire, UK, durchgeführt.

### Reinigung der Antikörper

In einem 1-Schritt Verfahren wurden aus den Antiseren, die beginnend mit dem vierten Monat nach der Immuniserung gewonnen worden waren, die Peptid-spezifischen Antikörper präpariert. Dazu wurden zunächst die Peptide PAV15 und MPGC22 an SulfoLink Gel gekoppelt (s. Arbeitsanleitung "SulfoLink Kit" Firma PIERCE, Rockford, IL, USA). Dabei wurden zur Kopplung je 5 mg Peptid pro 5 ml Gel angeboten.

Die Affinitätsreinigung von Peptid-spezifischen Antikörpern aus Schaf Antiseren gegen die Peptide wurde wie folgt durchgeführt:

Die Peptid-Säulen wurden zunächst dreimal im Wechsel mit je 10 ml Elutionspuffer (50 mM Citronensäure, pH 2.2) und Bindungspuffer (100 mM Natriumphosphat, 0.1% Tween, pH 6.8) gewaschen. 100 ml der Antiseren wurden über 0,2 µm filtriert und mit dem vorhandenen Säulenmaterial versetzt. Dazu wurde das Gel quantitativ mit 10 ml Bindungspuffer aus der Säule gespült. Die Inkubation erfolgte über Nacht bei Raumtemperatur unter Schwenken. Die Ansätze wurden quantitativ in Leersäulen (NAP 25, Pharmacia, entleert) überführt. Die Durchläufe wurden verworfen. Anschließend wurde mit 250 ml Bindungspuffer proteinfrei (Proteingehalt des Wascheluats < 0,02 A280 nm) gewaschen. Auf die gewaschene Säulen wurde Elutionspuffer gegeben, und es wurden Fraktionen à 1 ml gesammelt. Von jeder Fraktion wurde der Proteingehalt mittels der BCA-Methode (s. Arbeitsanleitung Firma PIERCE, Rockford, IL, USA) bestimmt.

Fraktionen mit Proteinkonzentrationen > 0.8 mg/ml wurden gepoolt. Nach Proteinbestimmung der Pools mittels der BCA-Methode ergaben sich Ausbeuten von 27 mg für den anti-PAV15 Antikörper und 35 mg für den anti- MPGC22 Antikörper.

### Markierung

Über NAP-5 Gelfiltrationssäulen (Pharmacia) wurden 500 µl des gereinigten anti-MPGC22 Antikörpers s.o.) in 1 ml 100 mM Kalium-Phosphatpuffer (pH 8,0) nach Arbeitsanleitung umgepuffert. Die Proteinkonzentationen der Antikörperlösung wurden mit 100 mM Kalium-Phosphatpuffer (pH 8,0) auf 1,5 mg/ml eingestellt.

Zur Chemilumineszenzmarkierung wurde der Antikörper wie folgt weiterbehandelt: 67 µl der Antikörperlösung wurden mit 10 µl MA70-Akridinium-NHS-Ester (1 mg/ml; Firma HOECHST Behring) versetzt und 15 Minuten bei Raumtemperatur inkubiert. Dann wurden 423 µl 1 M Glycin zugesetzt und weitere 10 Minuten inkubiert. Anschließend wurde der Markierungsanastz über eine NAP-5 Gelfiltrationssäule (Pharmacia) in 1 ml Laufmittel A (50 mM Kaliumphosphat, 100 mM NaCl, pH 7.4) nach Arbeitsanleitung umgepuffert und dabei von niedermolekularen Bestandteilen befreit. Zur Abtrennung letzter Reste nicht an Antikörper gebundenen Labels wurde eine Gelfiltrations-HPLC durchgeführt (Säule: Waters Protein Pak SW300). Die Probe wurde aufgetragen und bei einer Flußrate von 1 ml/min mit Laufmittel A chromatographiert. Mit einem Duchflußphotometer wurden die Wellenlängen 280 nm und 368 nm gemessen. Das Absorbtionsverhältnis 368 nm/280 nm als Maß für den Markierungsgrad des Antikörpers betrug am Peak 0.10 +/- 0.01. Die monomeren Antikörper enthaltenden Fraktionen (Retentionszeit 8-10 min) wurden gesammelt und in 3 ml 100 mM Natriumphosphat, 150 mM NaCl, 5% Bovines Serum Albumin, 0.1% Natrium Azid, pH 7.4, gesammelt.

### Kopplung

Bestrahlte 5 ml Polystyrolröhrchen (Firma Greiner) wurden mit gereinigtem anti-PAV15 Antikörper wie folgt beschichtet: Der Antikörper wurde in 50 mM Tris, 100 mM NaCl, pH 7.8 zu einer Konzentration von 6.6 µg/ml verdünnt. In jedes Röhrchen wurden 300 µl dieser Lösung pipettiert. Die Röhrchen wurden 20 Stunden bei 22°C inkubiert. Die Lösung wurde abgesaugt. Dann wurde jedes Röhrchen mit 4.2 ml 10 mM Natriumphosphat, 2% Karion FP, 0.3% Bovines Serum Albumin, pH 6.5 befüllt. Nach 20 Stunden wurde die Lösung abgesaugt. Schließlich wurden die Röhrchen in einem Vakuumtrockner getrocknet.

### Durchführung und Auswertung des Immunoassays

Als Standardmaterial diente Peptid PAR35, das seriell in Pferdenormalserum (Firma SIGMA) verdünnt wurde. Den so hergestellten Standards wurden Konzentrationen gemäß der Peptideinwaage zugeschrieben.

Der Sandwichimmunoassay wurde wie folgt angesetzt: In die jeweiligen mit Antikörpern beschichteten Teströhrchen wurden 50 µl Standards bzw. Proben sowie 200 µl Assaypuffer (100 mM Natriumphosphat, 150 mM NaCl, 5% Bovines Serum Albumin, 0.1% unspez. Schaf IgG, 0.1% Natrium Azid, pH 7.4), enthaltend 1 Million RLU (relative light units) des MA70-markierten Antikörpers, pipettiert. Es wurde 2 Stunden bei 22°C unter Schütteln inkubiert. Dann wurde 4 x mit je 1 ml Waschlösung (0.1% Tween 20) pro Röhrchen gewaschen, abtropfen gelassen und die am Röhrchen gebundene Chemilumineszenz in einem Luminometer (Firma BERTHOLD, LB952T; Basisreagenzien BRAHMS AG) vermessen. Unter Verwendung der Software MultiCalc (Spline Fit) wurden die NT-proET-1 Konzentrationen der Proben an der Standardkurve abgelesen.

Analyt, der mit dem beschriebenen Assay gemessen werden kann, wird bezeichnet als N-terminales proEndothelin-1 (NT-proET-1).

### Klinische Wertigkeit (Beispiele, die Patienten mit Erkrankungen der Atemwege und/oder Lunge betreffen dienen als Referenz)

### Normalbereich

NT-proET-1 Konzentrationen wurden in Proben von gesunden Kontrollpersonen (n=200) bestimmt. Der Median lag bei 30,5 pmol/L, der kleinste gemessene Wert bei 2,0 der größte bei 53 pmol/L, die 95% Percentilen bei 16,3 bzw. 47,2 pmol/L.

### Herzinsuffizienz / Schweregrad

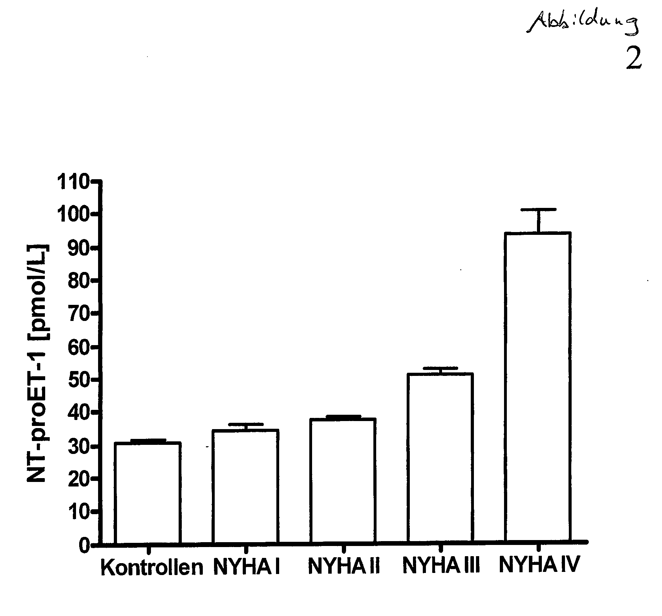

Es wurden NT-proET-1 Konzentrationen bei Patienten mit chronischer oder akut dekompensierter Herzinsuffizienz vermessen. NT-proET-1 Konzentrationen waren mit dem Schweregrad der Herzinsuffizienz assoziiert, insbesondere mit erhöhtem Schweregrad: Die Mediane der NT-proET-1 Konzentrationen bei den vier Schweregrad-Kategorien NYHA I-IV waren: 35,4, 37,3, 45,5 bzw. 83,9 pmol/L (s. Fig. 2).

### Chronische Herzinsuffizienz / Diagnose

Von einem Kollektiv von 316 Patienten mit chronischer Herzinsuffizienz sowie 200 gesunden Kontrollen wurden NT-proET-1 Werte bestimmt. Die Receiver-Operator-Characteristics Analyse ergab eine AUC von 0,72. Bei einem Cut-off-Wert von 49,7 pmol/L ergab sich eine Sensitivität von 34,3% bei einer Spezifität von 98%. Bei einem Cut-off-Wert von 47,2 pmol/L ergab sich eine Sensitivität von 38,5% bei einer Spezifität von 95%.

### Chronische Herzinsuffizienz / Prognose

Von einem Kollektiv von 316 Patienten mit chronischer Herzinsuffizienz wurden NT-proET-1 Werte bestimmt. Die Patienten wurden über einen mittleren Zeitraum von 360 Tagen beobachtet. In diesem Zeitraum starben 42 Patienten, 274 überlebten. Durch Receiver-Operator-Characteristics Analyse wurde der beste Cut-off-Wert (definiert als das größte Produkt aus Sensitivität und Spezifität) zur Prognose der Mortalität ermittelt: 51,3 pmol/L. Bei diesem Cut-off-Wert betrug die Sensitivität der Prognose 71,4%, die Spezifität betrug 74,5%. Die Likelihood Ratio zu versterben betrug bei einem Cut-off-Wert von 51,3 pmol/L: 2,8.

| | <51,3 pmol/L | >51,3 pmol/L |
|---|---|---|
| Überlebende | 204 | 70 |
| Tote | 12 | 30 |

### Akute Herzinsuffizienz / Diagnose

Von einem Kollektiv von 125 Patienten mit akuter Atemnot wurden NT-proET-1 Werte bestimmt. Von den 125 Patienten hatten 69 Patienten Herzinsuffizienz. Die Receiver-Operator-Characteristics Analyse für die Differentialdiagnose der Herzinsuffizienz ergab eine AUC von 0,72. Bei einem Cut-off-Wert von 111 pmol/L ergab sich eine Sensitivität von 10,2% bei einer Spezifität von 98%. Bei einem Cut-off-Wert von 90,5 pmol/L ergab sich eine Sensitivität von 17,5% bei einer Spezifität von 95%.

### Akute Herzinsuffizienz / Prognose

Von einem Kollektiv von 69 Patienten mit akut dekompensierter Herzinsuffizienz wurden NT-proET-1 Werte bestimmt. Die Patienten wurden über einen Zeitraum von 360 Tagen beobachtet. In diesem Zeitraum starben 21 Patienten, 48 überlebten. Durch Receiver-Operator-Characteristics Analyse wurde der beste Cut-off-Wert (definiert als das größte Produkt aus Sensitivität und Spezifität) zur Prognose der Mortalität ermittelt: 64 pmol/L. Bei diesem Cut-off-Wert betrug die Sensitivität der Prognose 66,6%, die Spezifität betrug 68,7%. Die Likelihood Ratio zu versterben betrug bei einem Cut-off-Wert von 64 pmol/L: 2,0.

| | | |
|---|---|---|
| | <64 pmol/L | >64 pmol/L |
| Überlebende | 33 | 15 |
| Tote | 7 | 14 |

### Myokardinfarkt / Prognose

Von 246 Patienten mit akutem Herzinfarkt wurden drei Tage nach Eintreten des Herzinfarkts Proben gewonnen und es wurde NT-proET-1 gemessen. Die Patienten wurden über einen Zeitraum von 60 Tagen beobachtet. In diesem Zeitraum hatten 220 Patienten kein adverses Ereignis, 26 starben oder wurden wegen Herzinsuffizienz rehospitalisiert. Durch Receiver-Operator-Characteristics Analyse (AUC = 0,70) wurde der beste Cut-off-Wert (definiert als das größte Produkt aus Sensitivität und Spezifität) zur Prognose der Mortalität bzw. Rehospitalisierung wegen Herzinsuffizienz ermittelt: 63,9 pmol/L. Bei diesem Cut-off-Wert betrug die Sensitivität der Prognose 65,4%, die Spezifität betrug 73,6%. Die Likelihood Ratio eines adversen Ereignisses betrug bei einem Cut-off-Wert von 63,9 pmol/L: 2.5.

| | | |
|---|---|---|
| | <63,9 pmol/L | >63,9 pmol/L |
| kein adverses Ereignis | 162 | 58 |
| Tot/Herzinsuffizienz | 9 | 17 |

### Pneumonie / Schweregrad und Prognose

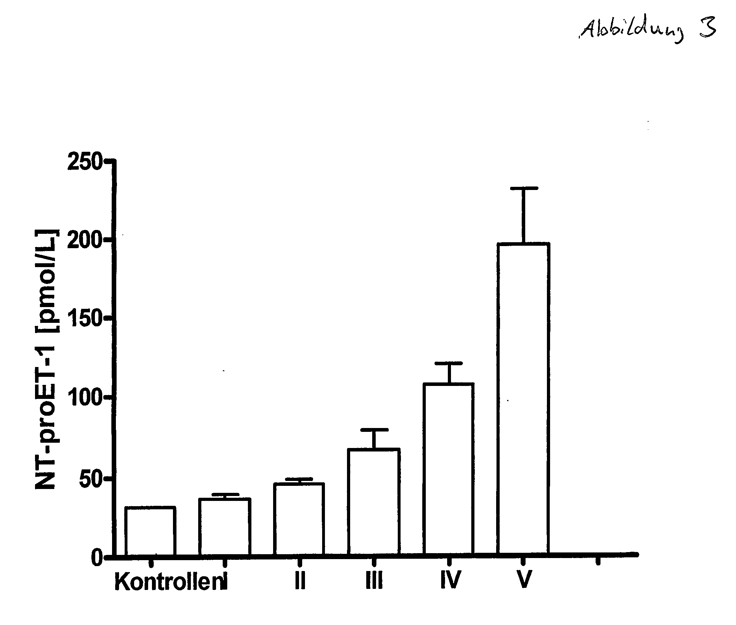

Von 142 Patienten mit ambulant erworbener Pneumonie wurden bei Aufnahme ins Krankenhaus Proben gewonnen und es wurde NT-proET-1 gemessen. Die Patienten wurden über einen Zeitraum von 70 Tagen beobachtet. In diesem Zeitraum starben 10 Patienten. NT-proET-1 Konzentrationen stiegen mit dem PSI (Pneumonia Severity Index), einem Score für den Schweregrad der Erkrankung (Abbildung 3) und waren im Median mit 53 pmol/L gegenüber gesunden Kontrollen (30,5 pmol/L) erhöht. Für die Prognose der Mortalität erbrachte die Receiver-Operator-Characteristics Analyse eine AUC von 0,89. Durch Receiver-Operator-Characteristics Analyse wurde der beste Cut-off-Wert (definiert als das größte Produkt aus Sensitivität und Spezifität) zur Prognose der Mortalität ermittelt: 77 pmol/L. Bei diesem Cut-off-Wert betrug die Sensitivität der Prognose 100%, die Spezifität betrug 81,2%. Die Likelihood Ratio der Mortalität betrug bei einem Cut-off-Wert von 77 pmol/L: 5.5.

| | <77 pmol/L | >77 pmol/L |
|---|---|---|
| kein adverses Ereignis | 108 | 24 |
| Tot | 0 | 10 |

Exacerbierte COPD. Von 53 Patienten mit chronisch obstruktiver Lungenerkrankung und gleichzeitiger Infektion der unteren Atemwege wurde NT-proET-1 gemessen. Mit im Median 47 pmol/L waren diese Patienten gegenüber gesunden Kontrollen (30,5 pmol/L) erhöht, lagen jedoch niedriger als Pneumonie Patienten (s.o. 53 pmol/L).

**Tabelle: Klassifikation der New York Heart Association (NYHA)**

| | |
|---|---|
| NYHA I | Keine körperliche Einschränkung. Alltägliche körperliche Belastung verursacht keine inadäquate Erschöpfung, Rhythmusstörungen, Luftnot oder Angina |
| | Pectoris. |
| NYHA II | Leichte Einschränkung der körperlichen Belastbarkeit. Keine Beschwerden in Ruhe. Erschöpfung, Rhythmusstörungen, Luftnot oder Angina pectoris bei alltäglicher körperlicher Belastung. |
| NYHA III | Höhergradige Einschränkung der körperlichen Leistungsfähigkeit bei gewohnter Tätigkeit. Keine Beschwerden in Ruhe. Erschöpfung, Rhythmusstörungen, Luftnot oder Angina pectoris bei geringer körperlicher Belastung. |
| NYHA IV | Beschwerden bei allen körperlichen Aktivitäten und in Ruhe. Bettlägrigkeit. |

### Beschreibung der Abbildungen:

Abbildung 1 zeigt die Aminosäuresequenz (AS) von preproEndothelin-1 mit den Abschnitten 1-17 Signalpeptid, 18-52 NT-proET-1, 53-73 reifes ET-1, 53-90 Big ET-1 (bzw. 74-90).
Abbildung 2:
   NT-proET-1 Konzentrationen in Abhängigkeit vom Schweregrad der Herzinsuffizienz. Dargestellt sind die Mittelwerte (+SEM) von gesunden Kontrollen (n=200), sowie von
   Herzinsuffizienzpatienten gruppiert nach NYHA Schweregrad (NYHA I: n=22, NYHA II: n=126, NYHA III: n=132, NYHA 4: n=17).
Abbildung 3:
   NT-proET-1 Konzentrationen in Abhängigkeit vom Schweregrad der Pneumonie. Dargestellt sind die Mittelwerte (+SEM) von gesunden Kontrollen (n=200), sowie von Pneumoniepatienten gruppiert nach Pneumonia Severity Index (PSI I: n=36, PSI II: n=44, PSI III: n=25, PSI IV: n=19 , PSI V: n=18).

## Patentansprüche

1. Verfahren zur in-vitro Diagnose und / oder Risikostratifizierung von Herzerkrankungen, **dadurch gekennzeichnet, dass** eine Bestimmung des freien Fragments 18-52 aus SEQ ID No. 1 (NT-proET-1) oder ein Fragment ausgewählt aus SEQ ID No. 2, SEQ ID No. 3 in einer Körperflüssigkeitsprobe von einem zu untersuchenden Patienten erfolgt.

2. Verfahren zur Diagnose und / oder Risikostratifizierung von Herzerkrankungen nach Anspruch 1, **dadurch gekennzeichnet, dass** die Herzerkrankungen ausgewählt sind aus akutem Koronarsyndrom, post-Myokardinfarkt, Herzinsuffizienz.

3. Verfahren zur Diagnose und / oder Risikostratifizierung nach einem der vorherigen Ansprüche, zur Identifizierung von Patienten mit erhöhtem Risiko oder/und einer ungünstigen Prognose von Herzerkrankungen.

4. Verfahren zur Diagnose und / oder Risikostratifizierung nach einem der vorherigen Ansprüche, wobei der Patient ein symptomatischer und / oder asymptomatischer Patient, insbesondere ein Notfallpatient ist.

5. Verfahren zur Diagnose und / oder Risikostratifizierung nach einem der vorherigen Ansprüche, zur Therapiesteuerung von Herzerkrankungen, insbesondere in der Intensivmedizin oder Notfallmedizin.

6. Verfahren zur Diagnose und / oder Risikostratifizierung von Herzerkrankungen nach einem der vorherigen Ansprüche zur Durchführung von klinischen Entscheidungen, insbesondere weiterführenden Behandlungen und Therapien mittels Arzneimitteln, insbesondere in der Intensivmedizin oder Notfallmedizin, einschließlich der Entscheidung zur Hospitalisierung des Patienten.

7. Verfahren zur Diagnose und / oder Risikostratifizierung von Herzerkrankungen nach einem der vorherigen Ansprüche zur Prognose, zur differentialdiagnostischen Früherkennung und Erkennung, zur Beurteilung des Schweregrades und zur therapiebegleitenden Verlaufsbeurteilung.

8. Verfahren nach einem der vorherigen Ansprüche,
**dadurch gekennzeichnet,**
**dass** zusätzlich eine Bestimmung mindestens eines weiteren Markers ausgewählt aus der Gruppe inflammatorischer Marker, cardiovaskulärer Marker, neurohormonaler Marker oder ischämischer Marker durchgeführt wird.

9. Verfahren nach einem der vorherigen Ansprüche, **dadurch gekennzeichnet, dass** der inflammatorische Marker aus mindestens einem Marker aus der Gruppe C-reaktives Protein (CRP), Cytokine, wie etwa TNF-alpha, Interleukine, wie etwa IL-6, Procalcitonin (1-116, 3-116) und Adhäsionsmoleküle, wie VCAM oder ICAM ausgewählt ist.

10. Verfahren nach einem der vorherigen Ansprüche, **dadurch gekennzeichnet, dass** der cardiovaskuläre Marker aus mindestens einem Marker aus der Gruppe Kreatinkinase, Myoglobin, Myeloperoxidase, natriuretisches Protein, insbesondere ANP (bzw. ANF), proANP, NT-proANP, BNP, proBNP, NT-proBNP oder jeweils eine Teilsequenz davon, kardiale Troponin, CRP sowie Kreislaufregulierende (Pro)Hormone, wie pro-Gastrin-Releasing Peptid (proGRP), pro-Endothelin-2, pro-Endothelin-3, pro-Leptin, pro-Neuropeptid-Y, pro-Somatostatin, pro- Neuropeptid-YY, pro-Opiomelanocortin, pro-Adrenomedullin (proADM), pro-Vasopressin (proAVP) oder jeweils eine Teilsequenz davon ausgewählt ist.

11. Verfahren nach einem der vorherigen Ansprüche, **dadurch gekennzeichnet, dass** der ischämische Marker aus mindestens einem Marker aus der Gruppe Troponin I und T, CK-MB ausgewählt ist.

12. Verfahren nach einem der vorherigen Ansprüche, **dadurch gekennzeichnet, dass** der neurohormonale Marker mindestens ein natriuretisches Protein ist, insbesondere ANP (bzw. ANF), proANP, NT-proANP, BNP, proBNP, NT-proBNP oder eine Teilsequenz davon ist.

13. Verfahren nach einem der vorherigen Ansprüche,
**dadurch gekennzeichnet,**
**dass** parallele oder simultane Bestimmungen der Marker durchgeführt werden.

14. Verfahren nach einem der vorherigen Ansprüche,
**dadurch gekennzeichnet,**
**dass** die Bestimmungen an mindestens einer Patientenprobe durchgeführt werden.

15. Verfahren nach einem der vorherigen Ansprüche,
**dadurch gekennzeichnet,**
**dass** die Bestimmungen an einem Analyseautomaten, insbesondere mittels eines Kryptors, durchgeführt werden.

16. Verfahren nach einem der vorherigen Ansprüche,
**dadurch gekennzeichnet, dass** die Bestimmungen mittels eines Schnelltests, insbesondere in Einzel- oder Multiparameterbestimmungen durchgeführt werden.

17. Verwendung des freien Fragments 18-52 aus SEQ ID No. 1 (NT-proET-1) oder ein Fragment ausgewählt aus SEQ ID No. 2, SEQ ID No. 3 zur Diagnose und / oder Risikostratifizierung von Herzerkrankungen und ggfs. weiterer Marker nach einem der Ansprüche 8 bis 12.

18. Verwendung eines Kits zur Diagnose und / oder Risikostratifizierung von Herzerkrankungen enthaltend Nachweisreagenzien zur Bestimmung des freien Fragments 18-52 aus SEQ ID No. 1 (NT-proET-1) oder ein Fragment ausgewählt aus SEQ ID No. 2, SEQ ID No. 3 und ggfs. weitere Marker nach einem der Ansprüche 8 bis 12 und Hilfsmittel.

## Claims

1. A method for *in* vitro diagnosis and/or risk stratification of heart diseases, **characterised in that** a determination of the free fragment 18-52 from SEQ ID No. 1 (Nt-proET-1) or a fragment selected from SEQ ID No. 2, or SEQ ID No. 3 is carried out in a bodily fluid sample from a patient to be examined.

2. The method for diagnosis and/or risk stratification of heart diseases according to Claim 1, **characterised in that** the heart diseases are selected from acute coronary syndrome, post-myocardial infarction, and heart failure.

3. The method for diagnosis and/or risk stratification according to one of the preceding claims for identifying patients having an increased risk and/or an unfavourable prognosis of heart diseases.

4. The method for diagnosis and/or risk stratification according to one of the preceding claims, wherein the patient is a symptomatic and/or asymptomatic patient, and in particular is an emergency patient.

5. The method for diagnosis and/or risk stratification according to one of the preceding claims for controlling the therapy of heart diseases, in particular in intensive care or emergency care.

6. The method for diagnosis and/or risk stratification of heart diseases according to one of the preceding claims for the execution of clinical decisions, in particular advanced treatments and therapies by means of drugs, in particular in intensive care or emergency care, including the decision to hospitalise the patient.

7. The method for diagnosis and/or risk stratification of heart diseases according to one of the preceding claims for prognosis, for early detection, and detection by differential diagnosis, for assessment of the degree of severity, and for assessment of the course of the disease concomitant with the therapy.

8. The method according to one of the preceding claims,
**characterised in that**,
in addition, a determination of at least one further marker selected from the group of inflammatory markers, cardiovascular markers, neurohormonal markers or ischaemic markers is carried out.

9. The method according to one of the preceding claims,
**characterised in that** the inflammatory marker is selected from at least one marker from the group of C-reactive protein (CRP), cytokines, such as TNF-alpha, interleukins, such as IL-6, Procalcitonin (1-116, 3-116) and adhesion molecules, such as VCAM or ICAM.

10. The method according to one of the preceding claims,
**characterised in that** the cardiovascular marker is selected from at least one marker from the group of creatine kinase, myoglobin, myeloperoxidase, natriuretic protein, in particular ANP, (or ANF), proANP, NT-proANP, BNP, proBNP, NT-proBNP or a partial sequence thereof, cardiac troponin, CRP and circulation-regulating (pro)hormones, such as pro-gastrin releasing peptide (proGRP), pro-endothelin-2, pro-endothelin-3, pro-leptin, pro-neuropeptide-Y, pro-somatostatin, pro-neuropeptide-YY, pro-opiomelanocortin, pro-adrenomedullin (proADM), pro-vasopressin (proAVP) or a partial sequence thereof.

11. The method according to one of the preceding claims,
**characterised in that** the ischaemic marker is selected from at least one marker from the group of troponin I and T, and CK-MB.

12. The method according to one of the preceding claims,
**characterised in that** the neurohormonal marker is at least one natriuretic protein, in particular ANP (or ANF), proANP, NT-proANP, BNP, proBNP, NT-proBNP or a partial sequence thereof.

13. The method according to one of the preceding claims,
**characterised in that**
parallel or simultaneous determinations of the markers are carried out.

14. The method according to one of the preceding claims,
**characterised in that**
the determinations are carried out on at least one patient sample.

15. The method according to one of the preceding claims,
**characterised in that**
the determinations are carried out using an automatic analysis system, in particular by means of a Kryptor.

16. The method according to one of the preceding claims,
**characterised in that** the determinations are carried out by means of a rapid test, in particular in individual parameter or multi-parameter determinations.

17. Use of the free fragment 18-52 from SEQ ID No. 1 (NT-proET-1) or of a fragment selected from SEQ ID No. 2, or SEQ ID No. 3 for diagnosis and/or risk stratification of heart diseases and optionally further markers according to one of Claims 8 to 12.

18. Use of a kit for diagnosis and/or risk stratification of heart diseases containing detection reagents for determining the free fragment 18-52 from SEQ ID No. 1 (NT-proET-1) or a fragment selected from SEQ ID No. 2, SEQ ID No. 3 and optionally further markers according to one of Claims 8 to 12 and auxiliary agents.

## Revendications

1. Procédé pour le diagnostic in vitro et/ou la stratification des risques de maladies cardiaques, **caractérisé en ce qu'**une détermination du fragment libre 18-25 de la SEQ ID n°1 (NT-proET-1) ou d'un fragment choisi parmi SEQ ID n°2, SEQ ID n°3 est effectuée pour un échantillon de liquide corporel d'un patient à examiner.

2. Procédé pour le diagnostic et/ou la stratification des risques de maladies cardiaques selon la revendication 1, **caractérisé en ce que** les maladies cardiaques sont choisies parmi le syndrome coronarien aigu, le post-infarctus du myocarde et l'insuffisance cardiaque.

3. Procédé pour le diagnostic et/ou la stratification des risques selon l'une des revendications précédentes, pour l'identification de patients avec un risque accru et/ou un pronostic défavorable de maladie cardiaque.

4. Procédé pour le diagnostic et/ou la stratification des risques selon l'une des revendications précédentes, dans lequel le patient est un patient symptomatique et/ou asymptomatique, en particulier un patient en situation d'urgence.

5. Procédé pour le diagnostic et/ou la stratification des risques selon l'une des revendications précédentes, pour le contrôle thérapeutique de maladies cardiaques, en particulier dans les soins intensifs ou la médecine d'urgence.

6. Procédé pour le diagnostic et/ou la stratification des risques de maladies cardiaques selon l'une des revendications précédentes, pour l'exécution de décisions cliniques, en particulier les traitements continus et les traitements à base de médicaments, en particulier dans les soins intensifs ou la médecine d'urgence, y compris la décision relative à l'hospitalisation du patient.

7. Procédé pour le diagnostic et/ou la stratification des risques de maladies cardiaques selon l'une des revendications précédentes, pour le pronostic, la détection précoce et la détection de diagnostics différentiels, pour l'évaluation du niveau de gravité et pour l'évaluation de l'évolution sous traitement.

8. Procédé selon l'une des revendications précédentes,
**caractérisé en ce que**
une détermination d'au moins un marqueur supplémentaire, choisi parmi le groupe comprenant un marqueur inflammatoire, un marqueur cardiovasculaire, un marqueur neurohormonal ou un marqueur ischémique, est en outre effectuée.

9. Procédé selon l'une des revendications précédentes,
**caractérisé en ce que** le marqueur inflammatoire est choisi à partir d'au moins un marqueur issu du groupe comprenant la protéine C-réactive (CRP), la cytokine, telle que l'alpha-TNF, l'interleukine, telle que l'IL-6, la procalcitonine (1-116, 3-116) et des molécules d'adhésion, telles que la VCAM ou l'ICAM.

10. Procédé selon l'une des revendications précédentes,
**caractérisé en ce que** le marqueur cardiovasculaire est choisi à partir d'au moins un marqueur issu du groupe comprenant la créatine kinase, la myoglobine, la myéloperoxydase, une protéine natriurétique, en particulier l'ANP (ou l'ANF), la proANP, la NT-proANP, la BNP, la proBNP, la NT-proBNP ou une séquence partielle de chacune de celles-ci, la troponine cardiaque, la CRP ainsi que les (Pro)hormones régulant la circulation, telles que le peptide de libération de la progastrine (proGRP), la pro-endothéline-2, la pro-endothéline-3, la pro-leptine, le pro-neuropeptide-Y, la pro-somatostatine, le pro-neuropeptide-YY, la pro-opiomélanocortine, la pro-adrénomédulline (proAMD), la pro-vasopressine (proAVP) ou une séquence partielle de chacun de ceux-ci.

11. Procédé selon l'une des revendications précédentes,
**caractérisé en ce que** le marqueur ischémique est choisi parmi un groupe comprenant la troponine I et T, CK-MB.

12. Procédé selon l'une des revendications précédentes,
**caractérisé en ce que** le marqueur neurohormonal est au moins une protéine natriurétique, en particulier l'ANP (ou l'ANF), la pro-ANP, la NT-proANP, la BNP, la proBNP, la NT-proBNP ou une séquence partielle de celles-ci.

13. Procédé selon l'une des revendications précédentes,
**caractérisé en ce que**
des déterminations parallèles ou simultanées des marqueurs sont effectuées.

14. Procédé selon l'une des revendications précédentes,
**caractérisé en ce que**
les déterminations sont effectuées pour au moins un échantillon de patient.

15. Procédé selon l'une des revendications précédentes,
**caractérisé en ce que**
les déterminations sont effectuées sur un dispositif d'analyse automatique, en particulier au moyen d'un Kryptor.

16. Procédé selon l'une des revendications précédentes,
**caractérisé en ce que** les déterminations sont effectuées au moyen d'un test rapide, en particulier dans les déterminations à un seul paramètre ou à paramètres multiples.

17. Utilisation du fragment libre 18-52 de SEQ ID n°1 (NT-proET-1) ou d'un fragment choisi parmi SEQ ID n°2, SEQ ID n°3 pour le diagnostic et/ou la stratification des risques de maladies cardiaques, et éventuellement d'autres marqueurs selon l'une des revendications 8 à 12.

18. Utilisation d'un kit pour le diagnostic et/ou la stratification des risques de maladies cardiaques contenant des réactifs de détection pour la détermination du fragment libre 18-52 de SEQ ID n°1 (NT-proET-1) ou d'un fragment choisi parmi SEQ ID n°2, SEQ ID n°3, et éventuellement d'autres marqueurs selon l'une des revendications 8 à 12 et d'autres moyens.
